(19) Europäisches Patentamt
European Patent Office
Office européen des brevets

(11) EP 1 591 542 A1

(12) EUROPEAN PATENT APPLICATION

(43) Date of publication:
02.11.2005 Bulletin 2005/44

(51) Int Cl.[7]: C12Q 1/68, C12Q 1/04

(21) Application number: 05014297.5

(22) Date of filing: 24.08.2000

(84) Designated Contracting States:
DE FR GB
Designated Extension States:
AL LT LV MK RO SI

(30) Priority: 25.08.1999 JP 23781899

(62) Document number(s) of the earlier application(s) in accordance with Art. 76 EPC:
00954993.2 / 1 231 280

(71) Applicant: National Institute of Advanced Industrial Science and Technology
Tokyo 100-8921 (JP)

(72) Inventors:
- Maruyama, Akihiko, c/o National Institute of Adv.
1-1 Higashi Tsukuba-shi Ibaraki 305-8561 (JP)
- Higashihara, Takanori, National Institute of Adv.
1-1 Higashi Tsukuba-shi Ibaraki 305-8561 (JP)
- Ishiwata, Hiroyuki
1-chome Minato-ku Tokyo 105-8401 (JP)
- Fujita, Tsunemi, NYK Logistics Techn. Inst. Co.Ltd
Chiyoda-ku, Tokyo 1000-0005 (JP)

(74) Representative:
Manitz, Finsterwald & Partner GbR
Postfach 31 02 20
80102 München (DE)

Remarks:
This application was filed on 30 - 06 - 2005 as a divisional application to the application mentioned under INID code 62.

(54) **Novel 16SrRNA gene data and probes**

(57) 16SrDNAs having a base sequence represented by any of SEQ ID NOS: 1 to 4. RNA or DNA probes of 10 to 50 bp in size which have a part of a base sequence represented by any of SEQ ID NOS: 1 to 4 and are hybridizable specifically with a petroleum-digesting bacterium belonging to the genus *Cycloclasticus.*

EP 1 591 542 A1

Printed by Jouve, 75001 PARIS (FR)

**Description**

Technical Field

The present invention relates to a method of detecting and quantitating a microorganism having a specific function and its gene from natural environment, novel 16SrRNA gene data, and use thereof.

Background of the Invention

Petroleum-degrading microorganisms inhabit in the sea. In an oil-polluted sea area where an oil spill accident has occurred, they act to degrade the oil and purify the sea into its natural state with a lapse of time. Hitherto, various treatment methods and techniques for the oil have been developed through elucidation of physico-chemical factors that are responsible for natural purification (self-purification) of the oil (R.P.J. Swannell, K. Lee and M. Mcdonagh, Microbiol. Rev., 60: 342-365, 1996). However, the constitution and fluctuation of the microbial community or consortium, responsible for natural purification has scarcely been elucidated. In this state, it is very difficult to assess the effectiveness or universality of the treatment technique. There is an attempt to promote cleanup of the polluted sea by addition of microbial inocula which can efficiently degrade petroleum under culture conditions, though such an artificially added microbial inocula has not yet been assessed sufficiently on its safety and effectiveness in natural environment.

It is required a great deal of labor and time to monitor pollutant-degrading microorganisms in an environment polluted with petroleum or harmful chemicals, because the degrading microorganism has to be counted by a culture method, and as well the microorganism has to be isolated from environmental samples by an enrichment culture or plate culture method, and the petroleum in the culture broth of isolates has to be analyzed to assess its biodegradability. It is also very important that among microorganisms inhabiting the natural environment, those that currently can be isolated and cultured as mentioned above are only 1% or less. Accordingly, in the conventional isolation and cultivation methods described above for monitoring petroleum- or harmful chemical-degrading bacteria, only approximately 1% of microorganisms in the target field can be monitored, and it is not possible to monitor most of microorganisms hard to be isolated involved in degradation of petroleum or harmful chemicals in a polluted field.

Recently, bioremediation utilizing a microorganism that can degrade such harmful chemicals is in the development stage for cleanup of soil or ground water polluted with harmful chemicals such as PCB, trichloroethylene, etc., in addition to petroleum [Tohru Kodama et al., edit. "Small living creatures preserving the earth - Environmental microorganisms and bioremediation", Gihodo Press, 1995; Osami Yagi, Application of Bioremediation to the aquatic environment, under the supervision of Japan's Fisheries Association [Yuzaburo Ishida and Akinori Hino, edit.]: Restoration of Environment by Biological Function - Possibility of Bioremediation in Fisheries, Koseisha Koseikaku, p. 9-21, 1996]. However, behavior of microbial community and degrading microorganisms in the process of bioremediation have not generally been elucidated.

The object of the present invention is to provide a method for elucidating a microorganism degrading pollutants such as petroleum or harmful chemicals which hold a predominant position in the field environment polluted with petroleum and harmful chemicals. Also provided are materials and the methods for detecting a microorganism degrading pollutants such as petroleum or harmful chemicals, which is essential to proceed the development of environmental cleanup and restoration techniques utilizing a microorganism, and microorganism producing a useful substance such as enzymes from the natural environment.

Disclosure of Invention

The present inventors have developed a novel method for monitoring a petroleum-degrading bacteria which comprises detecting and quantitating said bacterium from a sample of a petroleum-polluted field at the level of genes using a combined technique comprising microplate MPN, direct PCR and DNA sequencing, and then analyzing a dominant level of the petroleum-degrading bacteria through comparing the total number of the microorganism by means of a direct counting technique using a microscope. According to this method, it is possible to overcome the past problems since no isolation of petroleum-degrading bacteria is necessary and monitoring is possible for petroleum-degrading bacteria even if they were hardly isolable (not easy to be isolated). Moreover, according to the above-mentioned method, without using a plate culture method, a microorganism with specific function such as microorganisms degrading pollutants such as harmful chemicals or microorganism producing useful substances such as enzymes, is detected and quantitated at the molecular or cellular level. Thus, the invention is useful as a novel monitoring method or a novel screening method of microorganism producing useful substances applicable to the analysis of a dominant level and behavior of these microorganisms with specific function in an environment.

In addition, the present inventors have succeeded in elucidating the molecular-phylogenic characteristics from the nucleotide sequence data of 16S rDNA of the petroleum-degrading bacterium, which holds a predominant position in the field environment, by determining nucleotide sequence of the 16S rDNA of a microorganism holding a predominant position in the oil-polluted sea water which was collected on January 15, 1997, by the above methods after the oil spill accident by the tanker Nakhodka, which occurred on January 2, 1997 in the Sea of Japan. The inventors have also succeeded in developing DNA probes by which the above microorganism can be labeled specifically, based on the nucleotide sequence data thus obtained, because microorganisms can be detected at the molecular and cellular level without using any culture method.

The present invention was completed on the basis of these findings.

The invention is outlined as follows.

(1) A method for detecting and quantitating a microorganism having a specific function and its gene from the natural environment, which comprises:

1) the step of subjecting a microorganism-containing samples collected from the natural environment to serial dilution, then incubating it under the conditions which the microorganism having a specific function can grow, and then counting the grown microorganism having a specific function, and in parallel with these operations, counting the total number of microorganisms in the above microorganism-containing samples, and simultaneously counting the total number of heterotrophic microorganisms, and estimating the dominant level of the microorganism having a specific function in the natural environment from the ratio of the number of the microorganism having a specific function to the total number of microorganisms and/or of heterotrophic microorganisms;
2) the step of extracting DNA from the microorganism in culture broth of the highest dilution ratio at which the growth of the microorganism is judged as positive, and amplifying specific gene regions using said DNA as templates, followed by cloning;
3) the step of examining the difference of the gene regions thus cloned, and determining the nucleotide sequences thereof; and
4) the step of identifying the microorganism having a specific function inhabiting in the natural environment from the nucleotide sequences data thus determined.

(2) A method as described in the above item (1), wherein the number of the microorganism having a specific function and the total number of heterotrophic microorganisms is counted by an MPN method, the total number of the microorganisms is counted by a direct microscopic counting method, and the growth of the microorganism having a specific function is judged by observation under a microscope.
(3) A method as described in the above item (1) or (2), wherein the microorganism having a specific function is a microorganism which degrades a specific chemical substance.
(4) A method as described in the above item (3), wherein the specific chemical substance is a harmful chemical substance.
(5) A method as described in the above item (3), wherein the specific chemical substance includes petroleum and petroleum components.
(6) A method for assessing the function of a microbial community in the natural environment by analyzing succession of the microorganism existing predominantly in the natural environment using the method as described in the item (1) or (2).
(7) A method for analyzing and assessing a polluted environment using the method as described in the item (1) or (2).
(8) A method for analyzing and evaluating an environment polluted by harmful chemicals using the method as described in the item (3) or (4).
(9) A method for analyzing and evaluating an oil-polluted environment using the method as described in the item (5).
(10) A method as described in the item (1) or (2), wherein the microorganism having a specific function is a microorganism producing a useful enzyme.
(11) A 16S rDNA having the nucleotide sequence represented by any of SEQ ID NOS: 1 to 4.
(12) An RNA or DNA probe with the length of from 10 to 50 bases which has a part of the nucleotide sequence represented by any of SEQ ID NOS: 1 to 4 and is hybridizable specifically with a petroleum-degrading bacterium belonging to the genus *Cycloclasticus.*
(13) An RNA or DNA probe as described in the item (12), wherein the part of the nucleotide sequence represented by any of SEQ ID NOS. 1 to 4 is selected from the group consisting of the nucleotide sequences represented by SEQ ID NOS: 5, 6 and 7.
(14) An RNA or DNA probe as described in the item (12) or (13), which is used in detection or quantification of a petroleum-degrading bacterium belonging to the genus *Cycloclasticus.*
(15) An RNA or DNA probe as described in the item (14), wherein the petroleum-degrading bacterium belonging to the genus *Cycloclasticus* is *Cycloclasticus pugetii* or its closely related species.

(16) An RNA or DNA probe as described in the item (12) or (13), which is used in screening a petroleum-degrading bacterium belonging to the genus *Cycloclasticus.*

(17) An RNA or DNA probe as described in the item (16), wherein the petroleum-degrading bacterium belonging to the genus *Cycloclasticus* is *Cycloclasticus pugetii* or its closely related species.

(18) A method for detecting and quantitating a petroleum-degrading bacterium belonging to the genus *Cycloclasticus* using the RNA or DNA probe as described in the item (12) or (13).

(19) A method as described in the item (18), wherein the petroleum-degrading bacterium belonging to the genus *Cycloclasticus* is *Cycloclasticus pugetii* or its closely related species.

(20) A method for screening a petroleum-degrading bacterium belonging to the genus *Cycloclasticus* using the RNA or DNA probe as described in the item (12) or (13).

(21) A method as described in the item (20), wherein the petroleum-degrading bacterium belonging to the genus *Cycloclasticus* is *Cycloclasticus pugetii* or its closely related species.

(22) A method for identifying a petroleum-degrading bacterium belonging to the genus *Cycloclasticus* by means of DNA/DNA or DNA/RNA hybridization using an RNA or DNA probe homologous to any of SEQ ID NOS: 1 to 4 or as described in the item (12) or (13).

(23) A method as described in the item (22), wherein the petroleum-degrading bacterium belonging to the genus *Cycloclasticus* is *Cycloclasticus pugetii* or its closely related species.

The invention provides a method for detecting and quantitating a microorganism having a specific function and its gene from the natural environment, which comprises: 1) the step of subjecting a microorganism-containing samples collected from the natural environment to serial dilution, then incubating it under the conditions which the microorganism having a specific function can grow, and then counting the grown microorganism having a specific function, and in parallel with these operations, counting the total number of microorganisms in the above microorganism-containing samples, and simultaneously counting the total number of heterotrophic bacteria, and estimating the dominant level of the microorganism having a specific function in the natural environment from the ratio of the number of the microorganism having a specific function to the total number of microorganisms and/or of heterotrophic bacteria; 2) the step of extracting DNA from the microorganism in a culture broth of the highest dilution ratio at which the growth of the microorganism is judged as positive, and amplifying specific gene regions using said DNA as templates, followed by cloning; 3) the step of examining the difference of the gene regions thus cloned, and determining the nucleotide sequences thereof; and 4) the step of identifying the microorganism having a specific function inhabiting in the natural environment from the nucleotide sequences data thus determined. In this method, it is essential to count the total number of microorganisms in order to know the size of a microbial community (the size of population) in a sample from the target natural environment. This counting has a very important significance in the step of estimating the dominant level of a specifically functional microorganism in the sample.

Herein, the "natural environment" refers to the biosphere on the earth, including hydrosphere such as sea and lakes and marshes, rivers, waste water treatment environment; geosphere such as soil, underground, ground under the bottom of the sea; and atmosphere such as the surface over the earth, that is, all of the microorganism-inhabitable environment on the earth.

The "microorganism having a specific function" refers to a microorganism inhabiting in the natural environment, of which the function has been characterized by means of culture or genetic analyses. For example, such a microorganism is exemplified by those which can degrade harmful chemicals such as petroleum, organochlorine compounds, e.g., trichloroethylene, PCB, dioxin, etc., endocrine disrupting substances (alkylphenol, bisphenol A, phthalic acid ester, etc.), organomercury, cyanogen compounds, organotin compounds, and the like, and which can produce useful substances such as degradative enzymes, e.g., chitinase, lipase, cellulase, xylanase, lignin-degrading enzyme, etc., or a variety of antibiotics.

The "gene of a microorganism having a specific function" means genetic information that is possessed by the above-mentioned microorganism having a specific function. For example, nucleotide sequence data on which the microorganism having a specific function can be characterized in genes involved in expression of the function and taxonomic standard genes such as ribosomal RNA gene (rDNA), topoisomerase gene, etc., are exemplified.

"Detection and quantification of a microorganism having a specific function and its gene" means that the above-mentioned microorganism having a specific function or its gene is specifically detected and quantified (counted). When the microorganism having a specific function is detected and quantified, it can be detected specifically on the basis of the presence of growth depending on the availability of substrates used in the culture. Thus, serial dilution of the sample prior to the incubation allows counting (quantification) of the microorganism. Also, qualitative analyses (identification of microorganisms and detection of specific genes) are allowed by analyzing the genes or cells of the microorganisms taken out from the culture broth which permitted counting.

That is, the gene is extracted and its nucleotide sequence is decoded with a sequencer to analyze the molecular phylogeny and homology, by which the identification of the microorganism and the detection of specific gene can be attained. Such serial-dilution-sorting-out culture reduce a multiplication error which becomes a problem of the usual PCR and permit an exact and quantitative analysis of the gene. In addition, a DNA/RNA probe or antibody specific to the microorganism having a specific function allows detection of the presence of said microorganism at a cellular level or molecular level (in a case of a sample prepared by disruption of the cells) using a detection technique through hybridization or a labeled material (fluorescence, enzyme, etc.) on the antibody. Therefore, in the course of such qualitative analysis, when the presence of a target material such as the nucleotide sequence of gene or an antigen in the sample is confirmed, the presence can be quantified by counting the number of the microorganism having the target material in the same way as in the above-mentioned microorganism having a specific function.

In the above-mentioned method, first, a microorganism-containing samples collected from the natural environment is serially diluted and incubated under the conditions in which the microorganism having a specific function can grow.

The "microorganism-containing samples collected from the natural environment" refers to that collected from the microorganism-inhabiting biosphere on the earth, for example, a sample such as water, soil, bottom mud, or rock collected from atmosphere, sea, lakes and marshes, rivers, soil, underground, ground under the bottom of the sea, etc. , as well as a sample in which microorganisms coexist or symbiotically live, such as microalga, macroalga, zooplankton, various animals and plants, etc. are exemplified.

Serial dilution of the microorganism-containing sample may be achieved, for example, as follows. When the microorganism is supposed to coexist or symbiotically live with other organisms or form a large conglomerate, the sample is first applied, for example, to treatment with a vortex mixer for several minutes or to ultrasonic washing for about 1 minute prior to the serial dilution. When the sample is solid, this is carried out in a sterilized initial diluent. This operation is made with observation and confirmation of dispersion under a microscope to freely disperse the microorganisms in the sample. Subsequently, the microorganism-containing sample is serially diluted with a sterilized diluent. For example, when the sample is diluted on a 10-fold serial dilution on the 10 ml scale, a sterilized diluent (for example, artificial or natural sea water in a case of a sample derived from the sea; distilled water in a fresh-water sample; physiological saline in a sample derived from animals or plants; or a culture medium used in respective culture) 9 ml each is distributed into sterilized test tubes, into which the microorganism-containing sample 1 ml each is added and shaken/agitated well. This is defined as the primary dilution sample (dilution ratio: $10^1$-fold, $10^{-1}$-fold sample). Subsequently, 1 ml of this primary dilution sample is added to 9 ml of fresh sterilized diluent and shaken/agitated well. This is defined as the secondary dilution sample (dilution ratio: $10^2$-fold, $10^{-2}$-fold sample). This operation is then repeated n times to give the n-order dilution samples (dilution ratio: $10^n$-fold, $10^{-n}$-fold sample). Thus, in 10-fold serial dilution, the ratio of the sample to the diluent may be in 1:9.

The respective serially diluted solutions are incubated under the conditions in which the microorganism having a specific function grows. For example, when the microorganism is a petroleum- or harmful chemical substance-degrading microorganism, the respective serially diluted solutions may be incubated in a culture medium containing petroleum or harmful chemicals as carbon sources. Herein, the "culture" or "incubation" means growing and multiplying microorganisms existing in the serially diluted solution, and may be carried out under the conditions where the temperature, humidity, pH, the medium components such as the kind and amount of nutrients are controlled.

After termination of the culture, the number of the multiplied microorganism having a specific function is counted. The counting of the microorganism may be achieved, for example, by the MPN method using a liquid medium (Ushio Shimizu: Measurement of the Number of General Heterotrophic Bacteria by the MPN method; An Investigation Manual for Coastal Environment II - Water Quality and Microorganisms; Edited by Japan's Society of Oceanography, Koseisha Koseikaku, p. 281, 1990) and/or by the plate counting method using a solid medium solidified with agar or silica gel. In this connection, when a general aerobic microorganism is targeted as a microorganism having a specific function, the culture medium may be prepared according to the usual method without gas replacement to achieve the culture. Alternatively, in the culture medium or the test tubes or vessels in which the medium is placed, air is replaced with nitrogen gas, etc., and the culture is carried out under conditions containing no oxygen. Thus, it is possible to target various anaerobic microorganisms for counting. Among these methods, the MPN method is preferred since the detection sensitivity is higher than that of the other methods.

More specifically, 3 or 5 incubation tubes containing 9 ml of the culture medium are provided for the respective serial dilutions of the above sample. Depending on the number of microorganism in the sample, approximately 4- to 6-fold diluted sample is provided. When the number of the microorganism is expected to be large, the sample diluted in advance may be used in counting. In the MPN method, the dilution ratio becomes 10 times. To these 3 or 5 incubation tubes, 1 ml each of the serially diluted samples containing the above microorganism is added. These tubes are incubated at an optimum temperature for the microorganism, for example, 20°C for a certain period of time, and the growth of the microorganism may be judged by turbidity of the culture medium. From the results, the number of the microorganism in the sample is calculated according to the MPN counting table (3-tube or 5-tube method).

When the number of a petroleum-degrading or harmful chemical-degrading bacterium is counted as a microorganism having a specific function by means of MPN, the counting can be achieved by adding petroleum or a harmful chemical substance as the sole carbon source to the above-mentioned culture medium. In a case of microorganisms producing a useful enzyme such as chitinase, the number can be counted by employing a substrate for the target enzyme (e.g., chitin for chitinase) as carbon source of the culture medium.

In the above method, though the growth of microorganism is usually judged based on the presence of turbidity of the microorganism caused by growth, it can also be judged by means of change of the medium composition with growth (e.g., decrease of pH or substrate concentration, increase of the product, etc.). In the sample of high dilution ratio, growth of the microorganism or change of the medium composition is sometimes poor depending on the medium components or culture conditions employed. In such a case, it is desired to observe the presence of the microorganism in the sample of culture medium directly under a microscope for judgement. The direct observation under a microscope may be carried out according to the direct microscopic counting method (*vide infra).* In this case, however, since the purpose is to judge the presence of the microorganism in the sample, it is sufficient if the significant number of the cells can be detected and confirmed in comparison with that of the control to which no sample is added.

Among the above-mentioned samples of which the growth is judged positive to a specific substrate, that of the highest dilution ratio contains theoretically 1 - 9 cells as the cell number of the microorganism(s) having a specific function immediately after the dilution, i.e., before starting the incubation. The number of the kind of microorganism(s) contained is also 1 - 9. Accordingly, even though isolation by a conventional technique using an agar plate medium is impossible, in the liquid culture medium of the highest dilution ratio at which the growth has been judged positive, there is much more in number of the microorganism(s) by the growth. When this sample is intended to analyze at a DNA level, it is an advantage that erroneous problems can be reduced at the time of conducting PCR. In practice, it is an advantage that a gene analysis technique (*vide infra)* permits estimation of the kind of microorganism(s) efficiently in high precision, and at the same time affords the data on the cell number and the kind of the microorganism(s) existing in the collected initial sample.

In parallel with a series of the above-mentioned procedures including serial dilution, incubation and counting of the microorganism(s) having a specific function, the total number of the microorganisms in the above microorganism-containing sample is counted, and concurrently the total number of heterotrophic microorganisms are counted.

The "total number of the microorganisms in the microorganism-containing sample" refers to the total cell number of the microorganisms existing in the sample, and practically it is represented as the cell density (the cell number per unit volume).

The total number of the microorganisms may be counted, for example, directly by using a hemocytometric plate on which the cells are fixed with, e.g., formalin, without staining, or by using a flow cytometer for counting the cells stained with various DNA fluorochromes. More precisely, it can be done by a direct microscopic counting method.

Among these methods, the direct microscopic counting method is the most reliable technique as a method for counting the total number of the environmental microorganisms, wherein as a method using a DNA specific staining agent, an acridine orange method (J.E. Hobbie et al., Appl. Environ. Microbiol., 33: 1225-1228, 1977) or a DAPI method (K.G. Porter and Y.S. Feig, Limmol. Oceanogr., 25: 943-948, 1980). When the cells are not stained, there is a high risk that particles other than microorganisms abundantly existing in the environmental sample would be counted. Moreover, when the counting is achieved mechanically after staining, there is a high possibility that suspectedly stained particles would be counted because the microbial cells are not recognized directly by staining. Accordingly, the direct microscopic counting method is most preferred in view of convenience and reliability.

The following indicates a specific working example of a direct microscopic counting method. The sample immediately after collection is fixed with addition of neutral formalin (approximately 2% final concentration) and preserved in a refrigerator. The sample is divided into small portions, to each of which is added a DAPI (4',6-diamidino-2-phenylindole) solution at the final concentration of 0.5-5 μg/ml to stain the cells for about 5 minutes. The stained microbial cells are then filtered through a Nuclepore filter of 0.2μm in pore size stained with a dark pigment to collect on the filter. This filter is removed with a pair of forceps and placed on a slide glass on which one drop of emulsion oil for a fluorescent microscope has been placed in advance, so that the filter surface becomes upwards. Then, one drop of the emulsion oil is dropped on the filter, on which a cover glass is placed. Thus prepared sample on which additional one drop of emulsion oil is placed is observed and counted under an epi fluorescence microscope of an oil immersion type. The total number of microorganisms may be calculated from the following equation.

Total number of microorganisms (cells/ml) = (Mean cell number per square × Filtration area on the filter)/(Filtrated amount of the aqueous sample (ml) × The area of square

Herein, the square means the size of a square grid visual field observed through a micrometer which is inserted into the eye lens.

The "heterotrophic microorganisms" refer to microorganisms for which organic compounds produced by other organisms are essential as cellular components during growth.

The counting of the total number of heterotrophic microorganisms may be carried out, for example, by the agar plate method using a solid culture medium or by the MPN method using a liquid medium. When the microorganism having a specific function is counted by the MPN method, it is appropriate to count the heterotrophic microorganisms by the MPN method, too. When an aqueous sample from the general natural environment is subjected to the counting of the heterotrophic microorganisms, the MPN method is better than the plate method in view of the detection sensitivity since the viable count of microorganisms is usually larger in the former method than in the latter.

Specifically, when the heterotrophic microorganisms are counted by the MPN method, a sample is incubated in a culture medium for the heterotrophic microorganisms (e.g., 1/2TZ medium as shown in Example 1) for a certain period of time as mentioned above. The growth of the microorganism is judged from turbidity of the culture medium, and in the subsequent procedure the total number of heterotrophic microorganisms in the sample is calculated from the resultant data referring to the MPN counting table.

Subsequently, the dominant level of the microorganism having a specific function in the natural environment is estimated from the ration of the microorganism having a specific function to the total number of microorganisms and/or the total number of heterotrophic microorganisms. Specifically, the same samples collected from the same natural environment at the same time are used as the target samples. The dominant level may be estimated by using as a denominator the total number of the microorganisms obtained by the direct microscopic counting method and as a numerator the number of the microorganism having a specific function obtained by the MPN method. In this situation, theoretically the number of the microorganism having a specific function exceeds never the total number of the microorganisms counted by recognition of the respective cells, and the dominant level can always be estimated within a range of 0-100%, accordingly. Alternatively, in place of the total number of microorganisms, the total number of heterotrophic microorganisms obtained by the MPN method may be put in the denominator to obtain the ratio of the predominantly existing microorganism having a specific function to the total number of heterotrophic microorganisms. Moreover, plural samples are collected from the same natural environment at the same time, and the number of the microorganisms is measured for the respective samples. Thus, the statistical treatment such as calculation of the mean values and standard deviation can be made in order to improve reliability of the dominant level of the microorganism having a specific function in the target natural environment.

In addition, from the microorganism in the culture medium of the highest dilution ratio at which the growth of the microorganism having a specific function is judged positive, DNA are extracted.

The extraction of DNAs from microorganisms may be carried out according to the known methods (e.g., a method as described in M.G. Murray and W.F. Thompson, Nucleic Acids Research, 8: 4321-4325, 1980).

The specific gene domains are then multiplied and cloned using the extracted DNA as a template.

The "specific gene domains" include, for example, 16S rDNA as well as ribosomal RNA genes such as 5S, 18S, 23S rDNA, topoiomerase genes, elongation factor gene, genes for carbon dioxide-fixation enzymes, and specifically functional genes for enzymes involved in the substrate specificity of the microorganisms.

The multiplication and cloning of the specific gene domains may be carried out according to the known methods (e.g., for the multiplication, a method as described in J. Sambrook et al. , Molecular cloning: a laboratory manual, second edition, Cold Spring Harbor Laboratory Press, New York, pp. 14.1-14.35, 1989; for the cloning, a method as described in D. Kaufman and G. Evans, Bio Techniques, 9: 304-406, 1990). In practice, several ten samples of clones are selected at random, and it is confirmed whether the samples contain the full length of the target specific gene domain by a gel electrophoretic analysis. For example, when the full length of the multiplied specific gene domain is 2 kbp, it can be confirmed with a band around 2 kbp formed on the gel electrophoresis.

Subsequently, difference among the cloned gene domains is examined, and their nucleotide sequence is determined.

Specifically, the clone samples obtained as mentioned above, desirably 30 or more samples, are digested with some restriction enzymes such as EcoRI, ApaI, XbaI, etc., and subjected to a gel electrophoretic analysis to examine the difference of polymorphism (RFLP) patterns in the length of fragments digested with the restriction enzymes. At this stage, since the samples that have shown the different RFLP pattern are considered to have different nucleotide sequence data, the clone samples are roughly divided into some groups to exclude the same clone samples as much as possible for selection. Their nucleotide sequence is then determined with a DNA sequencer. In addition to the selection of the samples by the RFLP pattern analysis, they can also be examined in advance on the difference by a hybridization analysis using DNA probes (in this case, homology of the nucleotide sequence targeted by the probes). Thus, the samples of which the difference is large (low homology) can be selected as the targets for the nucleotide sequence analysis. As mentioned above, in the process of selection by the technique of the invention, theoretically at most 9 groups appear. If more than 9 samples of clones appear and if the difference is diverse, it could be considered that there is high possibility of misjudgment in the serial dilution/incubation counting, though possibility of base substitution in virtue of the enzymes which are possessed by the microorganism used in the cloning step cannot be denied. Thus, the samples of the higher dilution ratio can be used as the targets for the detection of predominant microorganisms.

Finally, the microorganism having a specific function inhabiting in the natural environment can be identified from the nucleotide sequence thus determined. Specifically, the nucleotide sequence data of some representative species over every taxon groups are obtained from the database of Gene Bank, EMBL or DDBJ, and subjected to the multiple alignment treatment using such a program as Clustal W (version 1.7, Des Higgins, 1997) or Se-Al (version 1, Andrew Rambaut, 1996)(rearrangement in which the insertion or deletion between the plural nucleotide sequences targeted for analysis is removed to form an assembly of the sequences showing the presence of reciprocal substitution). Then, they are subjected to the molecular phylogeny and homology analysis as mentioned below, and judged and identified which microorganism with specific function is most closely related to the known species from the molecular phylogenic relationship and homogenicity of the nucleotide sequence.

The microorganisms thus identified exist in samples collected from the natural environment. As mentioned above, however, if they are detected and identified in the highly diluted samples, a probability that they have existed predominantly in the natural environment would be very high. Moreover, it will be possible to indicate the dominant level as % more scientifically by determining in advance the total number of microorganisms or of heterotrophic microorganisms in the same samples collected from the natural environment as mentioned above. For example, in a sample collected in the oil-polluted field, a petroleum-degrading bacterium predominantly existing in the field is identified in the method as mentioned above and the number of it is compared to that of the total number of microorganisms or the total number of heterotrophic microorganisms to indicate its dominant level in %.

Herein, the "identification" refers to only the process of a molecular phylogenic analysis based on the nucleotide sequence data in the modern classification or identification method targeting for culturable microorganisms, wherein the molecular phylogenic identity or relationship is demonstrated in the gene of the microorganisms to be targeted for analysis and that of the relative microorganisms. As the molecular phylogenic analysis, the neighborhood-joining method (NJ method; N. Saitou and M. Nei, Mol. Biol. Evol., 4: 406-425, 1987) and/or the maximum-parsimony method (MP method; W.M. Fitch, Syst. Zool., 20: 406-419, 1971), maximum likelihood method (ML method; J. Felsenstein, J. Mol. Evol., 17: 368-376, 1981), etc., may be employed. In practice, the analysis by the NJ method can be achieved using a software of Clustal W (version 1.7, Des Higgins, 1997) or PHYLIP (J. Felsenstein, version 3.57c, 1995), and the MP analysis is done by that of PAUP* (D.L. Swofford, test version 4.0d63), the ML analysis is done by that of MOLPHY (version 2.3b; J. Adachi and M. Hasegawa, Computer science monographs, no. 28. Tokyo Institute of Statistical Mathematics, 1996). As a result of these molecular phylogenic analyses, when some of the clones obtained form a cluster of operational taxonomic unit quite different from that of the known species on the phylogenetic tree (when the bootstrap value indicating a probability of the reproducibility of divergence is less than 70-80% as an empirical standard), they are judged as a molecular phylogenically new group, and can be considered to be possibly a taxonomically new species.

In order to improve the precision of the identification in the above method of the invention, it is appropriate to include a step of homology analysis, etc. In this homology analysis, the regions of which the respective nucleotide sequences have been determined and are comparable each other are used among the target nucleotide sequence regions. Preferably, the number of the nucleotides in the whole sequence of the target gene is put in the denominator, and the number of the nucleotides that are completely identical therein is put in the numerator. Thus, the homology can be calculated as %, for example, using the Genentyx-MAC 8.0 analysis software (Genetic Information Processing Software Co.). Particularly, this is advantageous in view that it has been proposed as an international standard that, when the homology in the whole regions of 16S rDNA as targets is 97% or more, they are estimated as identical species each other (E. Stackebrandt and B.M. Goebel, Int. J. Syst. Bacteriol. 44: 846-849, 1994). On the other hand, even though the species is estimated as identical on the molecular phylogenic analysis and the homology analysis, when the homology estimated by the present process is less than 100%, it suggests that there are a certain variation of the nucleotide sequence and its subspecies in the same species. This means that these can become important reference data on secondary work in which a species-specific nucleotide sequence is selected from the new nucleotide sequence data or the existing data on the nucleotide sequence database to design the probe.

The microorganisms having a specific function may be those capable of degrading a specific chemical substance, for example, petroleum and petroleum components, organochlorine compounds such as trichloroethylene, PCB, dioxin, etc., endocrine disputing substances (alkylphenol used in detergents, bisphenol A as raw material for polycarbonate resins, phthalic acid ester as plasticizer for plastics, etc.), harmful chemicals such as organomercury, cyanogen compounds, organotin compounds, and the like. The major hydrocarbons contained in petroleum (in the broad sense, usually called crude oil) are roughly classified into saturated hydrocarbons and aromatic hydrocarbons depending on their chemical structure. The former is further classified into paraffins including n-paraffins and branched paraffins, and cycloparaffins (naphthene) including monocyclic and polycyclic cycloparaffins. The latter is classified into monocyclic and polycyclic aromatic hydrocarbons (Takanori Higashihara, Gekkan Kaiyo (Monthly Journal of Sea), Vol.30, no.10, 613-621, 1998). In general, such microorganisms degrading hydrocarbons contained in crude oil are called petroleum-degrading microorganisms (J.G. Leahy and R.R. Colwell, Microbiol. Rev., 54: 305-315, 1990; R.M. Atlas and R.Bartha, Adv. Microbiol. Ecol., 12: 287-338, 1992).

The microorganisms having a specific function may be those producing a particular useful enzyme, for example, chitinase, lipase, cellulase, xylanase, lignin-degrading enzyme, and the like.

According to the above-mentioned method, it is possible to evaluate the function of microbial community in the natural environment by analyzing the succession of the microorganisms existing predominantly in the natural environment. Herein, the "function of microbial community" refers to the function performed as whole by the multiple types of microorganisms inhabiting in the target natural environment. Herein, the term species of microorganisms preferably refer to those of the ordinary classification unit, but it may be one functional unit in an experiment, for example, a functional unit for a PCB-degrading bacterial group. Therefore, the function possessed by only one microorganism having a specific function is not called the function of microbial community. Additionally, the interaction between different species of microorganisms in a microbial community is considered to be as strong as it may be called symbiosis, or it is considered merely existing, but it is difficult to specify individually all of them. So, the total function by the whole microorganisms found in the sample is called the function of microbial community.

In practice, there is no microorganism that alone has all of the so far known microbial functions. The microorganisms found therein have respectively a peculiar niche (ecological position) on various factors influencing on the growth such as nutrient or oxygen, and most of them are considered to have a weak mutual relationship. The existence of this function of a microbial community can easily be estimated from the fact that no similar growth is observed under the conditions using the same non-sterilized natural seawater, for example, even in the microorganism which grows well under conditions using sterilized seawater.

With respect to decomposition of petroleum, in addition to microorganisms degrading aliphatic hydrocarbons and aromatic hydrocarbons, many other microorganisms are involved in the further process of degradation of intermediate metabolites of these hydrocarbons to carbon dioxide. Additionally, it is considered that other microorganisms coexist and supply a trace amount of essential nutrients such as vitamins to these microorganisms. Thus, it can be considered that multiple types of microorganisms work in cooperation to convert petroleum into inorganic and non-toxic materials. In this case, when certain degradation products can serve as growth inhibitors, it is considered that coexistence of a microorganism that can utilize the degradation products would increase the efficiency of degradation. Thus, the microbial community as whole would manifest a high degrading function.

Specifically, the dominant level(s) of one or multiple types of microorganism(s) having a specific function in a microbial community, for example, aerobic hydrocarbon-degrading bacteria, PCB-degrading bacteria, protein-degrading bacteria, glucose-assimilating bacteria, and anaerobic sulfate-reducing bacteria or methanogenic bacteria, can be examined by aforementioned method. Thus, succession of each microorganism having a specific function can be analyzed through investigation of the changes over time in the dominant level. As a result, for example, when the dominant level of a sulfate-reducing bacterium rises in the coastal surface water at some time, it can be evaluated that the whole microbial population has potentially high sulfate-reducing ability at that time. It is estimated as a cause that such a bacterium has been transferred from the ordinary anaerobic inhabiting environment prior to collection of the sample due to factors such as fling-up of sediments on the sea bottom by a typhoon or tectonic movement, or inflow of anaerobic waste water.

Moreover, the polluted environment can be analyzed and evaluated according to the above-mentioned method. For example, when the target microorganism having a specific function is a heterotrophic bacterium predominantly existing specifically in waste water of a certain pulp factory, if its dominant level increases at that time, the environment could be judged to be polluted with this waste water in high probability. If a change of the dominant level is monitored for a long period in order to grasp a periodic or seasonal change of the succession, it will be possible to estimate whether its change is an unexpected incident or whether its load is an artificial event.

In addition, the environment polluted with harmful chemicals can be analyzed and evaluated according to the above-mentioned method. For example, when the target microorganism having a specific function is a PCB-degrading bacterium, if its dominant level increases at that time, the environment could be judged to be polluted with PCB in high probability, and the microbial community as the whole could be judged increasing its PCB-degrading function. Moreover, if a change of the dominant level is monitored for a long period in order to grasp a periodic or seasonal change of the succession, it will be possible to estimate whether its change is an unexpected incident or whether its load is an artificial event, e.g., inflow of industrial waste water.

In addition, the oil-polluted environment can be analyzed and evaluated according to the above-mentioned method. For example, when the target microorganism having a specific function is that resulting from petroleum components, e.g., a tetradecane- or anthracene-degrading bacterium, if its dominant level increases at that time, the environment could be judged to be polluted with petroleum in high probability, and the microbial community as the whole could be judged increasing its petroleum-degrading function. Moreover, if a change of the dominant level is monitored for a long period in order to grasp a periodic or seasonal change of the succession, it will be possible to estimate whether its change is an unexpected incident or whether its load is an artificial event, e.g., a ship accident.

The above-mentioned method can be applied to detection, quantification and screening of microorganisms producing useful substances such as useful enzymes, antibiotics, etc. For example, chitin, lipids, lignin, cellulose, xylan, etc., used as a carbon source in a culture medium are effective in growth of the microorganisms producing enzymes which degrade said carbon sources (e.g., chitinase, lipase, cellulase, xylanase, etc.), of which the gene can be detected and quantified. By growing a microorganism on a ordinary liquid culture medium composed of peptone and yeast extract, etc., it is possible to examine whether the product of said microorganism contains a useful substance such as antibiotics in order to detect and quantify the microorganism producing a useful substance.

Recently, the nucleotide sequences of the genes producing said useful substances such as enzymes have been elucidated and their database established. The present method, accordingly, allows a search of useful substances by elucidating a gene information of the microorganism existing predominantly in the sample and comparing the resulting nucleotide sequence with that of the existing gene database relative to a useful substance such as an enzyme. Moreover, it is also possible to grasp quantitatively the number of the microorganism that has a nucleotide sequence of the gene producing an objective useful substance such as an enzyme. This is very important for detection, quantification and screening of a microorganism producing a useful substance from nature.

In detection, quantification and screening of microorganisms producing useful substances such as enzymes, hitherto, a great deal of labor was required in order to isolate many microorganisms from various samples as isolation source, wherein the samples were subjected to an enrichment culture or agar plate method on a culture medium containing an enzyme substrate such as cellulose, chitin, as a sole carbon source, and the isolate was then examined for productivity of the useful substance.

As described above, the invention provides a method for allowing quantitative detection and analysis of microorganisms having the nucleotide sequence of gene producing a useful substance such as an enzyme from a microbial community in the natural environment, even though the microorganism is difficult to isolate by a plate culture method. The method of the present invention, accordingly, is more efficient and convenient than the so far known methods, and very profitable as a method for detection, quantification and screening of microorganisms producing a useful substance, which have not been targeted because of difficult isolation in plate culture.

According to the above-mentioned method, the gene data of 16S rDNA having a nucleotide sequence of SEQ ID NOs: 1 - 4 was obtained from the surface seawater of the coastal region of Mikuni-cho, Fukui Pref., at which a large quantity of heavy oil was cast ashore by an oil spill accident which occurred at the Sea of Japan on January 2, 1997. From this gene data, it was found that the petroleum-degrading bacterium existing predominantly in that environment was *Cyloclasticus pugetii* or its closely relative species. That is, as described in Example below, a petroleum-degrading bacterium that existed predominantly in polluted site was selected from a microbial population in the oil-polluted sea area where the oil spill accident has occurred by the MNP counting method with liquid culture medium containing C-heavy oil as the sole carbon source. Then, a DNA was extracted from the cultured cells of the selected petroleum-degrading bacterium, which DNA was used as a template to carry out PCR with a proper primer (for example, a primer corresponding to the conserved region of 16S rDNA). The PCR liquid product was then subjected to electrophoresis to give 16S rDNAs having SEQ ID NOs: 1 - 4, of which the nucleotide sequences were determined with an autosequencer. Using this gene data, the petroleum-degrading bacterium exisiting predominantly in the area was analyzed by the above-mentioned molecular phylogenic or homology method and identified to be *Cyloclasticus pugetii* or its closely relative species.

On the basis of the nucleotide sequence data of SEQ ID NOs: 1 - 4, an RNA and DNA probes can be designed so as to be applicable to a variety of utilities. The nucleotide sequence and length of the probe may be selected optionally depending on the utilities. For example, by a FISH (fluorescence in situ hybridization) method, in order to detect or quantitate a microorganism belonging to the genus *Cycloclasticus* in a sample (for example, water collected from the sea, river, lake and marsh in the area polluted with oil), particularly, *Cyloclasticus pugetii* or its closely relative petroleum-degrading bacterium, or to screen a microorganism belonging to the genus *Cycloclasticus,* particularly, *Cyloclasticus pugetii* or its closely relative petroleum-degrading bacterium, from many microbial flora, it is appropriate to design a probe which has 10-50 bases, preferably 15-25 bases in length, corresponding to the region selected from the nucleotide nos. 823-853 of the nucleotide sequences of SEQ ID NOs: 1 - 4 (in the nucleotide sequence of 16S rDNA in *Escherichia coli,* the region of nucleotide nos. 829-866 from the 5'-terminal position (numbering system)). The followings are exemplified as the probes.

(1) 5'-GGAAACCCGCCCAACAGT-3' (Cyclopug 829-846*, 18mer)(SEQ ID NO: 5)

3'-CCTTTGGGCGGGTTGTCA-5'

(2) 5'-TGCACCACTAAGCGGAAACC-3' (Cyclopug 847-866*, 20mer) (SEQ ID NO: 6)

3'-ACGTGGTGATTCGCCTTTGG-5'

(3) 5'-GGAAACCCGCCCAACAGTTGCACCACTAAGCGGAAACC-3' (Cyclopug 829-866*, 38mer)(SEQ ID NO: 7)

(wherein *(number) indicates the position(numbering system) from the 5'-terminal in the 16S rDNA nucleotide sequence of *Escherichia coli* (H.F. Noller and C.R. Woese, Science, 212: 403-411, 1981))

(wherein *(number) indicates the position (numbering system) from the 5'-terminal in the 16S rDNA nucleotide sequence of *Escherichia coli* (H.F. Noller and C.R. Woese, Science, 212:403-411, 1981))

The nucleotide sequence of the probe (3) is derived from the nucleotide sequences of the probes (1) and (2) by lateral conjugation. In this case, however, it should be cautious because there is a possibility of self-conjugation of the probes.

The probes may be synthesized in the known method, for example, the phosphoramide method or triester method. Alternatively, they may be synthesized by means of a DNA autosynthesizer.

The probes may be labeled with an isotope ($^{32}P$, $^{35}S$, etc.), fluorescent dye (biotin/avidin, digoxigenin/anti-digoxigenin-rhodamine, fluorescein-isothiocyanate (FITC), Lucifer Yellow CH, rhodamine 123, acridine orange, pyronin Y, ethidium bromide, propidium iodide, ethidium homodimer, BOBO-1, POPO-1, TOTO-1, YOYO-1, carboxyfluorescein diacetate (CFDA), fluorescein diacetate (FDA), carboxyfluorescein diacetate-acetoxymethyl ester (CFDA-AM), 5-cyano-2,30-ditolyl tetrazolium chloride (CTC), tetramethylrhodamine isothiocyanate (TRITC), sulforhodamine 101 acid chloride (Texas Red), Cy3, Cy5, Cy7, 2-hydroxy-3-naphthoic acid-2'-phenylanilide phosphate (HNPP), etc.), antigenic substance such as digoxigenin, and the like. When labeled with an antigenic substance, the probe may be detected by chemiluminescence or fluorescence generated through enzymatic decomposition of a substrate in an enzyme immunoassay technique.

Using the RNA or DNA probes of the invention, microorganisms belonging to the genus *Cycloclasticus,* particularly a petroleum-degrading bacterium *Cyloclasticus pugetii* or its closely relative species can be detected or quantitated or screened by a variety of hybridization methods (Southern blot method, Northern blot method, colony hybridization method, dot hybridization method, *in situ* hybridization (e.g., FISH method, etc.), and the like.

The followings describe as an example a method for detecting and quantitating a petroleum-degrading bacterium from the seawater collected at the site of an oil spill accident, using the DNA probe of the invention. The seawater is collected from the sea area of an oil spillage accident, and the microorganisms contained in the seawater are fixed on a filter (pore size 0.2µm) and hybridized with a DNA probe having the nucleotide sequence of SEQ ID NO: 5 labeled with a fluorescent dye. After washing out the probe, the bacterium (petroleum-degrading bacterium) which has hybridized with the DNA probe is detected and counted under a fluorescence microscope.

Using the DNA probe of the invention, a microorganism belonging to the genus *Cycloclasticus,* particularly a petroleum-degrading bacterium *Cyloclasticus pugetii* or its closely relative species can be screened from many microbial population by means of a colony hybridization technique, dot blot hybridization technique, flow cytometry, and the like.

In addition, it is possible to identify a microorganism belonging to the genus *Cycloclasticus* by means of DNA/DNA or DNA/RNA hybridization using an RNA or DNA probe which was designed based on the nucleotide sequences of SEQ ID NOs: 1-4, or the homology with the nucleotide sequence data of SEQ ID NOs: 1-4. Herein, "identification" refers to a homology calculation between the nucleotide sequences when the nucleotide sequence of the approximately whole region of the target gene was analyzed as in SEQ ID NOs: 1-4. And, when a probe having the taxonomically specific nucleotide sequence designed as described above (in this case, the nucleotide sequence specific to a microorganism belonging to the genus *Cycloclasticus,* particularly *Cycloclasticus pugetii* or its closely relative species) is used, it means a hybridization test between the nucleotide sequences contained in the target gene sample.

The calculation of homology is made as follows. The number of the nucleotide in the sequence of the target gene, preferably of the whole region in which the individual nucleotide sequences have been determined and are comparable each other, is put in the denominator, and the number of the nucleotide that are completely identical therein is put in the numerator. Thus, the homology can be calculated as %, for example, using the Genentyx-MAC 8.0 analysis software (Genetic Information Processing Software Co.).

The identification of a petroleum-degrading bacterium belonging to the genus *Cycloclasticus* based on the homology of the nucleotide sequence of SEQ ID NOs: 1-4 may be made according to the ordinary standard for judgement of a species from the nucleotide sequence of 16S rDNA. That is, when the homology is 97% or less on the approximately whole region of 16S rDNA, it may be judged to be a different species tentatively (E. Stackebrandt and B.M. Goebel, Int. J. Syst. Bacteriol., 44: 846-849, 1994).

For example, the homology of the nucleotide sequences of 16S rDNA in a certain microorganism is examined in comparison with those of SEQ ID NOs: 1-4 for calculation with an analysis software such as Genentyx-MAC 8.0 (vide supra). As a result, when the value is 97% or more, it is judged to be the same species, and when less than 97%, judged a different species.

In some classified groups, however, even though the homology is 97% or more, they have been judged different species. For example, it is known that the homology between *Vibrio cholerae* and *Vibrio mimicus* is 98.9-99.4% (Shimizu and Tsukamoto, Classification and Identification of Marine Bacteria, Marine Microorganisms and Biotechnology, Edited by Ushio Shimizu. Gihodo Press, pp.1-24, 1991). In addition, in a study for marine bacteria of the *Vibrio* family, it has been estimated that a group having 99.3% or more of the homology is within the range of one species, and a group having 97% or more is within one genus (Shimizu and Tsukamoto, vide supra). Accordingly, it should be considered that the identification of the homology based on the standard of 97% or more is tentative, and the relationship between the species and the homology in the target taxon should be examined in advance prior to application of this standard.

Specifically, since only one species of *Cycloclasticus pugetii* has been accepted in the genus *Cycloclasticus* bacteria, the homology of 97% tentatively accepts the identification that the bacterium is of said species. As clearly seen from the above example of the genus *Vibrio,* in said genus for which further study is required, the standard of about 97% leaves still more the possibility that said bacterium might belong to a different species.

The petroleum-degrading bacteria of the genus *Cycloclasticus* may be identified as follows by means of DNA/DNA or DNA/RNA hybridization using the RNA or DNA probe.

The hybridization technique used in the identification includes the dot blot hybridization technique (H.E.N. Bergmans and W.Gaastra, New Nucleic Acid Techniques (J.M. Walker ed.) ; Methods in Molecular Biology, vol. 4, Clifton, Humana press, pp. 385-390, 1983), colony hybridization technique (M. Grustein and D.S. Hogness, Proc. Nat. Acad. Sci. USA, 72: 3961-3965, 1975), Southern blot hybridization technique (E.M. Southern, J. Mol. Biol., 98: 503-507, 1975), fluorescent in situ hybridization (also called whole cell hybridization) technique (R.I. Amann et al., Microbiol. Rev., 59: 143-169, 1995), and the like. The DNA/RNA sample used in these hybridization analyses may be prepared from microorganisms according to the known methods as described in the respective literatures.

Even though any of the hybridization techniques is used, it is important that strict hybridization conditions between the probe used and the sample of standard strain having the target gene nucleotide sequence should be determined in advance, for example, according to the method as describe by D.A. Stahl and R. Amann (Development and application of nucleic acid probes; In: Nucleic acid techniques in bacterial systematics; E. Stackebrandt and M. Goodfellow (ed.); John Wiley and Sons, West Sussex, pp. 205-248, 1991), wherein the conditions such as temperature, the concentration of formamide in the buffer solution, etc. are estimated, and ideally when the complementarity does not satisfy 100%, the conditions under which the probe is liberated in the process of hybridization and during the subsequent washing should be determined experimentally.

Under the hybridization conditions thus determined, hybridization is made for an unknown sample, using a target sample of genes from the target standard strains as described above or from the standard strains taxonomically far different from them. As a result, the intensity of radioactivity, fluorescence, or chemiluminescence (in a detection system of antigen-antibody-enzyme, the system can be selected by changing the enzyme substrate) generated by a radioactive element or fluorescent substance or antigen (in this case, utilizing the activity of an enzyme bound to the corresponding antibody) directly labeled to the probe, is measured and compared to that of the target sample. When the resulting intensity of hybridization of the unknown sample is equal to that of the target standard strain and recognized a great difference with a taxonomically far different strain (ideally, when the detected intensity is below the measuring limit), the unknown sample is judged to be the same species as or defined to be its closely relative species to the target microorganism.

In addition, for example, a species can be identified by PCR using the nucleotide sequences (DNA fragments) of the above probes as primers. That is, the target microbial cells to be identified are lysed, to which is added the DNA fragment having the nucleotide sequence of the above probe as a primer and multiplied by PCR. The PCR product is analyzed by electrophoresis, and if multiplication of 16S rDNA is confirmed, the target bacterium would have the gene portion complementary to the DNA fragment used. That is, the target bacterium is defined to be the same species as that having 16S rDNA containing the nucleotide sequence of SEQ ID NO: 1.

The microorganism of the genus *Cycloclasticus* detected, screened or identified according to the method of the invention, particularly *Cyloclasticus pugetii* or its closely relative petroleum-degrading bacterium, may be utilized as a microbial preparation (for example, petroleum-degrading microbial preparation for bioremediation agent).

In addition, the use of 16S DNA or the nucleotide sequence data of the probes of the invention allows elucidation of the behavior of petroleum-degrading bacteria in natural environment or under the conditions of oil treatment. It is also effective in development of an environmental repairing technology (bioremediation technology) such as promotion of the efficiency of oil degradation in oil-polluted sea area by addition of inorganic nutrients or by seeding of the petroleum-degrading bacteria.

The present specification includes the content as described in the specification and/or drawings of Japanese Patent Application No. 11-237818 that is a priority application of the present application.

Best Mode for Carrying Out the Invention

The invention will be described specifically by the following examples. These examples, however, serve to illustrate but are not intended to limit the scope of the invention.

Example 1

(1) Investigation of petroleum-degrading microorganisms in the sea area of a heavy oil spill accident

The surface seawater of the coastal region of Mikuni-cho, Fukui Pref., at which a large quantity of heavy oil was cast ashore by an oil spill accident which occurred at the Sea of Japan on January 2, 1997, was collected on January 15 immediately after the pollution. This was filtered through a plankton net (about 30µm in mesh), and the petroleum-degrading microorganisms in the surface seawater of the polluted area were counted by a microplate MPN (M-MPN) counting method. The M-MPN counting was carried out as follows:

> One point eight ml each of 1/10NP medium (100 mg $NH_4NO_3$, 2 mg ferric citrate, 2 mg $K_2HPO_4$, 800 ml aged seawater, 200 ml distilled water; pH 7.8) was added to wells on a 24-well microplate. Then, after seawater sample was diluted on 10-fold in each well containing the medium on this microplate, 10 µl each of n-tetradecane, kerosene or C-heavy oil as the sole carbon source was added to those wells. Diluted seawater sample in the well was incubated at 20°C. This MPN counting was carried out in triplicate. The growth was judged by comparing turbidity of the medium or change of the floating oil in each well with that in the control wells without inocula. In parallel, the total number of microorganisms was counted basically according to the Porter and Feig method (K.G. Porter and Y.S. Feig, Limnol. Oceanogr., 25: 943-948, 1980), as well as the number of heterotrophic bacteria was counted according to the M-MPN counting using a 1/2TZ medium (2.5 g polypeptone, 0.5 g Bacto-Yeast extract, 4.77 g HEPES, 900 ml Kester's artificial seawater, 100 ml distilled water; pH 7.5). Moreover, the surface seawater was collected according to the season from January 15, 1997 immediately after the accident for about 1 year. And the total number of microorganisms in the seawater was counted by the direct microscopic counting method, and the number of the heterotrophic bacteria or petroleum-degrading bacteria was examined by the MPN method.

As a result, it was found that the total number of microorganisms in the oil spill sea area was kept at a level

of $10^5$ cells/ml order over 1 year and there was no considerable variation, but the number of the petroleum-degrading bacteria was markedly changed. That is, the number of the n-tetradecane-, kerosene- or C-heavy oil-degrading bacteria was such a high value as about $10^3$-$10^4$ MPN/ml (the ratio (dominant level) of the number of the degrading bacteria to the total number of microorganisms: 1-10%), but it was markedly reduced to 10-$10^3$ MPN/ml (dominant level: 0.01-1%) after the lapse of about 2 months at the same area. On the other hand, the number of the heterotrophic bacteria was $10^4$ MPN/ml immediately after occurrence of the pollution, and about 2 months later approximately the same value, $10^4$-$10^5$ MPN/ml. From these results, it was judged that the microbial community immediately after the oil spill accident had much higher degrading function to oil components than in an ordinary state even at a lower water temperature of 12-13°C in winter.

(2) Selection of the petroleum-degrading bacteria predominantly existing in the oil spill sea area

Using the surface seawater collected on January 15, 1997 immediately after the above-mentioned accident, a sample containing a petroleum-degrading microorganism was collected from the well (a sample of growth-positive front) of the highest serial dilution ratio at which the growth of the microorganism was judged positive among the wells containing a 1/10NP medium which was added C-heavy oil and used in the M-MPN counting. This sample of microorganism is a liquid culture medium derived from the microbial community in the oil spill sea area by diluting up to the limit by 10-fold serial dilution. Therefore, the microorganism therein is considered the most predominant one in the oil spill sea area.

(3) Extraction of DNA from the selected petroleum-degrading microorganism

The sample of growth-positive front was centrifuged to collect the cells, which were suspended in 567 μl of TE buffer (10mM Tris-HCl, 1mM EDTA, pH 8.0). To this suspension was added 30 μl of 0.01% sodium dodecyl sulfate (SDS) and 3 μl of 20 mg/ml proteinase K solution, and the mixture was incubated at 37°C for 1 hour. Then, 100 μl of 5M NaCl was added, and the mixture was stirred well and incubated at 65°C for 10 minutes. After addition of 700 μl of chloroform/isoamyl alcohol mixture (24:1), the mixture was gently stirred and centrifuged to collect the supernatant (aqueous layer), to which was added an equivalent volume of phenol/chloroform/isoamyl alcohol mixture (20:24:1). The mixture was gently stirred and contrifuged to collect the supernatant (aqueous layer), to which was added 0. 6 volume part of isopropanol, and DNA was collected by centrifugation. The DNA was washed with 70% ethanol (-20°C), dried and dissolved in 100 μl of TE buffer.

(4) Cloning

Using the DNA recovered and purified in (3) as a template and the primers 27f and 1525r corresponding to the conserved region of 16S rRNA, 16S rRNA was amplified by PCR.

27f: 5'-AGAGTTTGATCCTGGCTCAG-3' (SEQ ID NO: 8)

1525r: 5'-AAAGGAGGTGATCCAGCC-3' (SEQ ID NO: 9)

This PCR product (16S rDNA), after confirmation of the presence by electrophoresis, was subjected to cloning with Original TA Cloning(r) kit (Invitrogen Co.).

(5) RFLP (restriction fragment length polymorphism) analysis

First, 40 clones were selected at random, and it was confirmed whether the full length of 16S rRNA was inserted into these clones. As a result, it was found that the full length of 16S rRNA was inserted into 17 clones. Next, these 17 clones were grouped by an RFLP (restriction fragment length polymorphism) analysis using 5 different kinds of restriction enzymes (*Eco*RI, *Hind*III, *Sal*I, *Xba*I and *Rsa*I) in order to grasp a rough tendency of population of petroleum-degrading bacteria which was considered to have the predominance in the oil-polluted sea area. As a result, 1 to 3 fragment patterns were obtained on the respective restriction enzymes. Finally, these clones were classified into 4 groups from the respective fragment patterns by the 5 kinds of restriction enzymes.

(6) Full sequencing

From the 4 groups classified by the RFLP analysis in (5), every one clone, totally 4 clones (CHO11-1-1, CHO11-1-2, CHO11-1-4, CHO11-1-34) were selected, and the whole nucleotide sequence of 16S rRNA was determined.

The sequence was determined with an auto-sequencer (ALFred DNA Sequencer, Pharmacia AB) using an RNA sequence kit (Thermo Sequenase TM fluorescent labeled primer cycle sequencing kit with 7-deaza-dGTP, Amersham Co.), and the nucleotide sequence of 16S rDNA was determined.

(7) Molecular phylogenic analysis

From the nucleotide sequence data of 16S rDNA of thus determined 4 clones, the presence of microorganisms having data similar to them was examined as preliminary search using a search engine Ribosomal Database Project (RDP) II (Illinois University). Subsequently, the similar sequences as well as the sequences of the representative strains of the respective taxon were obtained (download) from the database (GenBank, EMBL, DDBJ, etc.), and subjected to the multiple alignment treatment with the nucleotide sequence of 16S rDNA of the 4 clones using such a program as Clustal W *(vide supra)* or Se-Al (*vide supra*). For this alignment data (about 1500 nucleotide of sequence), then, the molecular phylogenic analysis by the NJ method (*vide supra)* and the bootstrap analysis by 100 repetitions were carried out using the PHYLIP program (*vide supra).*

As a result, it was found that the petroleum-degrading microorganism which existed predominantly in the surface seawater immediately after the oil spillage accident in the Sea of Japan on January 1997 is a bacterium belonging to the γ-subgroup of Proteobacteria and closely related to *Cycloclasticus pugetii (C. pugetii*). In addition, as a result of a homology analysis for the nearly full length of the nucleotide sequence of 16S rDNA, it was found that the 3 clones (CHO11-1-2, CHO11-1-4, CHO11-1-34) among the 4 clones have about 99% of homology (99.2%, 98.8%, 99.1%, respectively) to that of *C. pugetii* (GenBank No. U12624), while the other one clone (CHO11-1-1) showed lower homology (97.2%). In these 4 clones, accordingly, it was estimated that the sequences were derived from the same species as *C. pugetii* or its closely related species. The nucleotide sequences of 16S rDNA of these 4 clones (CHO11-1-1, CHO11-1-2, CHO11-1-4, CHO11-1-34) which were judged to be the same species as *C. pugetii* or its closely related species are shown in SEQ ID NOs: 1-4.

*Cycloclasticus pugetii* which was judged to be closely related to the above 4 clones was recently reported as a microorganism which was isolated from the sediment on the bottom of the sea polluted by polychlorinated biphenyl at the coastal region of the Pacific Ocean in USA and which had an aromatic hydrocarbon degrading capacity (S.E. Dyksterhouse et al., Int. J. Syst. Bacteriol. 45: 116-123, 1995). Therefore, the standard strain of this species (ATCC 51542) was obtained to examine. As a result, it was found that this microorganism is characterized in that it grows only on a culture medium containing such a specific organic substance as biphenyl and merely produces small colonies on plate culture involving such a substance, and is difficult to isolate on a usually plate culture medium for heterotrophic bacteria.

On the other hand, the nucleotide sequence data of 16S rDNA of the present invention was first discovered from the microbial sample collected from the oil-polluted seawater immediately after an oil spillage accident. And the sample has a quite different characteristic in the view that it was not collected from the sediment on the bottom of the sea nor from the sea area polluted by polychlorinated biphenyl. As mentioned above, this is supposed to be caused by that isolation of *C. pugetii* or its closely related species is very difficult. In the future, however, they will possibly be detected widely in the sea area of the world by such a method as described in the present invention.

Since the nucleotide sequences (SEQ ID NOs: 1-4) of 16S rDNA were obtained from an oil-polluted field through serially diluted liquid culture media containing C-heavy oil as the sole carbon source, they can be considered to be the nucleotide sequence data of the gene resulting from the microorganism which degrades hydrocarbons in C-heavy oil to grow. As mentioned above, it was shown that the microorganism that has said sequence data is molecular-phylogenically closely related to *Cycloclasticus pugetii* that has a capacity degrading aromatic hydrocarbons and two have a high homology each other. Therefore, microorganisms which have the nucleotide sequences of SEQ ID NOs: 1-4 and *C. pugetii* or its closely related microorganisms can be considered to be bacteria which have a function degrading hardly degradable polycyclic aromatic hydrocarbons. This means that the gene data of SEQ ID NOs: 1-4 are quite effective in examining the microbial flora at an oil spill field, or in analyzing the state of oil pollution or oil degradation based on the microbial flora or distribution density of the microorganisms having said gene data.

That is, recalcitrant polycyclic aromatic hydrocarbons in spilled crude oil or heavy oil remain unchanged in the polluted sea area for a longer period than readily decomposable aliphatic hydrocarbons. Therefore, the nucleotide sequence data of SEQ ID NOs: 1-4 resulting from microorganisms that might possibly degrade hardly decomposable polycyclic aromatic hydrocarbons to grow can be utilized as an effective indicator in monitoring the process of repairing and purifying oil pollution at the actual oil-polluted coastal area or polluted field. In addition, the nucleotide sequence data of SEQ ID NOs: 1-4, though supposed to be derived from *Cycloclasticus pugetii* or its closely related microorganisms, dose not have 100% homology with the known sequences but new sequence data. They, accordingly, are estimated very useful in preparing some probes specific to the species or groups of this taxon.

Moreover, it became clear that a series of the above-mentioned techniques, i.e., microplate MPN/direct PCR/sequencing techniques, are very effective in analyzing petroleum-degrading microorganisms predominantly existing in the sea area.

Example 2

(1) Selection of species specific sequences for *Cycloclasticus pugetii* (Probe design)

Using the results of the molecular phylogenic analysis performed in Example 1, the sequences specific to *C. pugetii* were selected taking the high-order structure of 16S rRNAs into account (the probes were designed). The followings indicate 2 kinds of the probes thus designed (Cyclopug 829-846 and Cyclopug 847-866).

- Probe Cyclopug 829-846

5'-GGAAACCCGCCCAACAGT-3' (829-846*, 18mer)(SEQ ID NO: 5)

(3'-CCTTTGGGCGGGTTGTCA-5')

- Probe Cyclopug 847-866

5'-TGCACCACTAAGCGGAAACC-3' (847-866*, 20mer)(SEQ ID NO: 6)

(3'-ACGTGGTGATTCGCCTTTGG-5')

Wherein * indicates the position (numbering system) from the 5'-terminal in the 16S rDNA nucleotide sequence of *Escherichia coli.*

(2) Probe check by the database

In order to confirm the specificity of the probes designed in (1), a probe-match was searched using the database (Ribosomal Database Project II, RDP). A search engine attached to RDP was used to search the miss match sequence under a condition of 2 nucleotides. As a result, it was confirmed that the two probes as designed matched only with 6 strains of the genus *Cycloclasticus* on RDP and were completely identical with no miss match base. In addition, both probes were confirmed not to self-conjugate. Among these 6 strains of the genus *Cycloclasticus,* two are *C. pugetii,* and the remaining four strains are unidentified or unaccepted strains for *Cycloclasticus* sp. For reference, the source of the 6 strains of the genus *Cycloclasticus* which matched on RDP is shown in Table 1.

Table 1.

| Source of the genus *Cycloclasticus* bacteria registered on RDP | | |
|---|---|---|
| Source | DataBase accession no. | Reference |
| *Cycloclasticus pugetii* str. PS-1(T)ATCC51542 | U12624 | 1 |
| *Cycloclasticus pugetii* str. PS-1(T)ATCC51542 | L34955 | 1 |
| *Cycloclasticus* sp. N3-PA321 | U57920 | 2 |

1. DYKSTERHOUSE et al., Int. J. Syst. Bacteriol., 43: 116-123, 1995
2. GEISELBECHT et al., Appl. Environ. Microbiol. , 62: 3344-3349, 1996

Table 1. (continued)

| Source of the genus *Cycloclasticus* bacteria registered on RDP | | |
|---|---|---|
| Source | DataBase accession no. | Reference |
| *Cycloclasticus* sp. G | AF093002 | 3 |
| *Cycloclasticus* sp. E | AF093003 | 3 |
| *Cycloclasticus* sp. W | AF093004 | 3 |
| *Cycloclasticus* sp. (*oligotrophus*) | AF148215 | 4 |

3. GEISELBECHT et al., Appl. Environ. Microbiol. , 64: 4703-4710, 1998

4. BUTTON et al., Appl. Environ. Microbiol., 64: 4467-4476, 1998

Herein, as the nucleotide sequence data of 16S rDNA of the genus *Cycloclasticus,* 6 strains in 7 cases are recorded on the RDP database. In this database, the species which was reported in IJSB (International Journal of Systematic Bacteriology) and IJSEM (International Journal of Systematic and Evolutionary Microbiology) and accepted formally is only *C. pugetii*, i.e., one genus and one species at present. In Table 1, *C. oligotrophus* is an unaccepted species, and difference from *C. pugetii* is not clear (the homology between both sequences is 99.3%).

From the above fact, this time it became clear that the two probes which were designed from the nucleotide sequences of 16S rDNA of microorganisms derived from an oil-polluted environment have a very high specificity for *Cycloclasticus pugetii* or its closely related species.

Example 3

It was confirmed that the probes thus designed hybridize specifically with the standard strain of *Cycloclasticus pugetii* ATCC51542 by the fluorescence *in situ* hybridization (FISH) method.

(1) Synthesis and labeling of probes

Probes Cyclopug 829-846 and Cyclopug 847-866 of which the specificity was confirmed by means of the database in Example 2 were synthesized with a DNA synthesizer and purified in a conventional manner. These probes Cyclopug 829-846 and Cyclopug 847-866 were labeled at the 5'-terminal with tetramethylrhodamine isothiocyanate (TRITC).

(2) Incubation and fixation of the strains provided

The standard strain of *Cycloclasticus pugetii* ATCC51542 was obtained from American Type Culture Collection (ATCC) and incubated in a 1/2TZ medium *(vide supra)* containing polychlorinated biphenyl at 20°C. As controls, 4 types of the standard strains, *Pseudomonas aeruginosa (P. aeruginosa), Bacillus marinus (B. marinus*), *Vibrio parahaemolyticus (V. parahaemolyticus*) and *Psychrobacter immobilis (Psy. immobilis*) were incubated under the conditions, i.e. , culture medium and temperature, recommended by the depositary authority of the strains. The respectively cultured cells were fixed with 3% (w/v) paraformaldehyde/3xPBS (24.0 g NaCl, 0.6 g KCl, 4.32 g $Na_2HPO_4$, 0.72 g $KH_2PO_4$) solution (pH 7.2-7.4). The cells were collected by centrifugation and washed with 3xPBS buffer. In addition, as a control probe, probe EUB338 (R.I. Amann et al. , J. Bacteriol. 172: 762-770, 1990) specific to the *Bacteria* domain in the molecular phylogeny was provided.

(3) Hybridization

*C. pugetii* and 4 types of the control strains preliminarily treated in (2) respectively were dropped on gelatin-coated slide glasses (0.1% gelatin, 0.01% $KCr(SO_4)_2$), and dried at room temperature to immobilized thereon. Then, the slide glasses were dehydrated with 50, 80 and 100% ethanol.

On the samples immobilized on the slide glasses, 15 μl of a hybridization buffer (0.9M NaCl, sodium sulfate buffer [pH 7.2], 0.5% sodium dodecyl sulfate [SDS], 5mM EDTA 1mg/ml Denhalt solution × 10 Poly(A)) was dropped, and the probe was added at a concentration of 5 ng/μl and stored for hybridization in a hybrichamber (humid state) at 45°C for 4.5 hours. After completion of the hybridization, the surface of the slide glasses was washed with 5 ml of a washing buffer (50mM sodium phosphate buffer [pH 7.0], 0.1% SDS, 0.9M NaCl). The glasses were immersed in 50 ml of the washing buffer at 42°C for 30 minutes, then washed with distilled water, and dried under air. Then, 1 - 5 μg/ml of 4',6-diamidino-2-phenylindole (DAPI) was added on the samples, and stained at room temperature for 5 minutes.

After washing with distilled water and air-drying, an anti-fading agent was dropped thereon, and covered with slide glasses for sealing. The slide glasses were observed under a fluorescence microscope (Zeiss, Oberkochen, Germany) under UV excitation, B excitation and G excitation.

Table 2 shows the results. In the probes Cyclopug 829-846 and Cyclopug 847-866 as designed this time, DAPI was bound universally to the DNAs in the respective cells by UV excitation, so that blue fluorescence resulting from DAPI could be observed in C. *pugetii* and in the 4 types of the control strains (*P. aeruginosa, B. marinus, V. parahaemolyticus* and *Phy. immobilis*). When the same field of vision was observed under G excitation, however, only *C. pugetii* emitted red fluorescence caused by TRITC labeled at the 5'-terminal of the probe because it had the sequence complementary to the probes Cyclopug 829-846 and Cyclopug 847-866.

Table 2.

| Effectiveness of the DNA probes specific to *Cycloclasticus pugetii* or its closely related species | | | |
|---|---|---|---|
| Standard Strain | Probe Tested | | |
| Tested | Cyclopug 829-846 | Cyclopug 847-866 | EUB338 |
| *Cycloclasticus pugetii* | ○ | ○ | ○ |
| *Pseudomonas aeruginosa* | × | × | ○ |
| *Bacillus marinus* | × | × | ○ |
| *Vibrio parahaemolyticus* | × | × | ○ |
| *Psychrobacter immobilis* | × | × | ○ |
| ○: Indicating occurrence of hybridization and emission of fluorescence in observation under a microscope<br>×: Indicating no occurrence of hybridization and unsuccessful observation under a microscope | | | |

On the other hand, in Probe EUB338 specific to *Bacteria* domain, *C. pugetii* and the 4 types of the control strains, all emitted green fluorescence caused by FITC which was labeled at the 5'-terminal of EUB338 even under observation by B excitation.

From the above fact, this time it was demonstrated that the two probes that have been designed from the nucleotide sequences of 16S rDNA of a microorganism isolated from an oil-polluted environment are practically very effective in specifically detecting *Cycloclasticus pugetii* or its closely related species without obstruction caused by the higher-order structure.

All of publications, patents and patent applications cited in the present specification are herein incorporated by reference.

Industrial Applicability

According to the invention, a technique for elucidating microorganisms existing predominantly in the natural environment (for example, a field environment polluted with petroleum, etc.) was provided.

The nucleotide sequence data of 16S rDNA analyzed by the technique of the invention are considered to be derived from a naturally occurring petroleum-degrading microorganism having a function predominant in an oil-polluted environment. Therefore, the nucleotide sequence data of the DNA probes which have been designed to allow the specific detection of the above microorganism based on the gene and gene data are very useful in elucidating the behavior of petroleum-degrading microorganisms in nature or under the conditions of oil treatment. The data are also effective in development of an environmental repairing technology (bioremediation technology) such as promotion of the efficiency of oil degradation in the polluted sea area by addition of inorganic nutrients or by seeding of the petroleum-degrading microorganisms.

According to the invention, a method for elucidating the behavior of microorganisms predominant in the natural environment (for example, environment polluted with petroleum or harmful chemicals) or degrading microorganisms in the process of bioremediation was provided.

Moreover, according to the invention, a method for selecting a microorganism or a microbial population having a specific function by liquid culture not through plate culture method for isolation of the microorganism is provided. Also provided is a method for analyzing the function and phylogenetic position of the microorganism at a gene level. The present invention, accordingly, is useful in detection, quantification and screening of microorganisms having said degrading function as well as so far hardly isolated microorganisms producing useful substances such as enzymes.

SEQ ID Table Free Text

SEQ ID NOS: 1-4 show the nucleotide sequences of 16S rDNAs.
SEQ ID NOS: 5-7 show the nucleotide sequences of the probes.
SEQ ID NOS: 8 and 9 show the nucleotide sequences of the primers.

## Claims

**1.** A 16S rDNA having the nucleotide sequence represented by any of SEQ ID NOS: 1 to 4.

**2.** An RNA or DNA probe with the length of from 10 to 50 bases which has a part of the nucleotide sequence represented by any of SEQ ID NOS: 1 to 4 and is hybridizable specifically with a petroleum-degrading bacterium belonging to the genus *Cycloclasticus*.

**3.** An RNA or DNA probe as claimed in Claim 2, wherein the part of the nucleotide sequence represented by any of SEQ ID NOS: 1 to 4 is selected from the group consisting of the nucleotide sequences represented by SEQ ID NOS: 5, 6 and 7.

**4.** An RNA or DNA probe as claimed in Claim 2 or 3, which is used in detection or quantification of a petroleum-degrading bacterium belonging to the genus *Cycloclasticus.*

**5.** An RNA or DNA probe as claimed in Claim 4, wherein the petroleum-degrading bacterium belonging to the genus *Cycloclasticus* is *Cycloclasticus pugetii* or its closely related species.

**6.** An RNA or DNA probe as claimed in Claim 2 or 3, which is used in screening a petroleum-degrading bacterium belonging to the genus *Cycloclasticus*.

**7.** An RNA or DNA probe as claimed in Claim 6, wherein the petroleum-degrading bacterium belonging to the genus *Cycloclasticus* is *Cycloclasticus pugetii* or its closely related species.

**8.** A method for detecting and quantitating a petroleum-degrading bacterium belonging to the genus *Cycloclasticus* using the RNA or DNA probe as claimed in Claim 2 or 3.

**9.** A method as claimed in Claim 8, wherein the petroleum-degrading bacterium belonging to the genus *Cycloclasticus* is *Cycloclasticus pugetii* or its closely related species.

**10.** A method for screening a petroleum-degrading bacterium belonging to the genus *Cycloclasticus* using the RNA or DNA probe as claimed in Claim 2 or 3.

**11.** A method as claimed in Claim 10, wherein the petroleum-degrading bacterium belonging to the genus *Cycloclasticus* is *Cycloclasticus pugetii* or its closely related species.

**12.** A method for identifying a petroleum-degrading bacterium belonging to the genus *Cycloclasticus* by means of DNA/DNA or DNA/RNA hybridization using an RNA or DNA probe homologous to SEQ ID NO: 1 or as claimed in Claim 2 or 3.

**13.** A method as claimed in Claim 12, wherein the petroleum-degrading bacterium belonging to the genus *Cycloclasticus* is *Cycloclasticus pugetii* or its closely related species.

European Patent Office

# EUROPEAN SEARCH REPORT

Application Number
EP 05 01 4297

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| T | MARUYAMA A ET AL: "Dynamics of microbial populations and strong selection for Cycloclasticus pugetii following the Nakhodka oil spill." MICROBIAL ECOLOGY, vol. 46, no. 4, November 2003 (2003-11), pages 442-453, XP002285704 ISSN: 0095-3628 (ISSN print) ----- | 1-13 | C12Q1/68<br>C12Q1/04 |
| A | BUTTON D K ET AL: "A small, dilute-cytoplasm, high-affinity, novel bacterium isolated by extinction culture and having kinetic constants compatible with growth at ambient concentrations of dissolved nutrients in seawater" APPLIED AND ENVIRONMENTAL MICROBIOLOGY, vol. 64, no. 11, November 1998 (1998-11), pages 4467-4476, XP002285764 ISSN: 0099-2240 * abstract * ----- | 1-13 | |
| X | GEISELBRECHT A D ET AL: "ISOLATION OF MARINE POLYCYCLIC AROMATIC HYDROCARBON (PAH)-DEGRADINGCYCLOCLASTICUS STRAINS FROM THE GULF OF MEXICO AND COMPARISON OF THEIR PAH DEGRADATION ABILITY WITH THAT OF PUGET SOUND CYCLOCLASTICUS STRAINS" APPLIED AND ENVIRONMENTAL MICROBIOLOGY, WASHINGTON,DC, US, vol. 64, no. 12, December 1998 (1998-12), pages 4703-4710, XP002934766 ISSN: 0099-2240 * abstract * ----- | 1-13 | TECHNICAL FIELDS SEARCHED (Int.Cl.7)<br>C12Q |
| | -/-- | | |

The present search report has been drawn up for all claims

| Place of search | Date of completion of the search | Examiner |
|---|---|---|
| Munich | 9 September 2005 | Loubradou-Bourges, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 05 01 4297

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GEISELBRECHT A D ET AL: "ENUMERATION AND PHYLOGENETIC ANALYSIS OF POLYCYCLIC AROMATIC HYDROCARBON-DEGRADING MARINE BACTERIA FROM PUGET SOUND SEDIMENTS" APPLIED AND ENVIRONMENTAL MICROBIOLOGY, WASHINGTON,DC, US, vol. 62, no. 9, September 1996 (1996-09), pages 3344-3349, XP002934768 ISSN: 0099-2240 * abstract * ----- | 1-13 | |
| X | DYKSTERHOUSE S E ET AL: "CYCLOCLASTICUS PUGETII GEN. NOV., SP. NOV., AN AROMATIC HYDROCARBON-DEGRADING BACTERIUM FROM MARINE SEDIMENTS" INTERNATIONAL JOURNAL OF SYSTEMATIC BACTERIOLOGY, SOCIETY FOR GENERAL MICROBIOLOGY, READING, GB, vol. 45, no. 1, January 1995 (1995-01), pages 116-123, XP002934767 ISSN: 0020-7713 * abstract * ----- | 1-13 | TECHNICAL FIELDS SEARCHED (Int.Cl.7) |

The present search report has been drawn up for all claims

2

| Place of search | Date of completion of the search | Examiner |
|---|---|---|
| Munich | 9 September 2005 | Loubradou-Bourges, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

SEQUENCE LISTING

<110> Director-General of Agency of Industrial Science and Technology Nishimatu Construction CO., LTD NYK LOGISTICS TECHNOLOGY INSTITUTE

<120> A method for detecting and quantifying bacteria having a specific function and the genes thereof from natural environment, novle 16 S rDNA gene information, and probes

<130> probe

<140> PCT/JP00/05711

<141> 2000-08-24

<150> JP P1999-237818

<151> 1999-08-25

<160> 9

<170> PatentIn version 3.1

<210> 1

<211> 1532

<212> DNA

<213> Cycloclasticus pugetii

<220>

<221> rRNA

<222> (1)..(1532)

<223>

<400> 1
agagtttgat catggctcag attgaacgct ggcggcatgc ctaacacatg caagtcgaac 60

ggaaacagaa tgcagcttgc tagcaggcgg tcgagtggcg gacgggtgag ttatgcatag 120

gaatccgccc gatagtgggg gacaacctcc tgaaaacgct gctaataccg cataatcccg 180

cgggggcaaa gacggggacc ttcgggcctt gctctaatgg atgagcctac aggggattag 240

gtagttggtg aggtaacggc tcaccaaggc aacgatccct agctggtttg agaggatgat 300
cagccacact gggactgaga cacggcccag actcctacgg gaggcagcag tggggaatat 360
tgcacaatgg aggaaactct gatgcagcaa tgccgcgtgt gtgaagaagg ccttagggtt 420
gtaaagcact ttcagtaggg aggaaaagtt taagggtaat aacccttagg ccctgacgtt 480
acctacagaa gaagcaccgg ctaactccgt gccagcagcc gcggtaatac ggagggtgca 540
agcgttaatc ggaattactg ggcgtaaagc gcgcgcaggc ggttaaacaa gtcagatgtg 600
aaagccccgg gctcaacctg ggaactgcat ttgaaactgg ctagctagag tgtggtagag 660
gagagtggaa tttcaggtgt agcggtgaaa tgcgtagata tctgaaggaa caccagtggc 720
gaaggcggct ctctggacca acactgacgc tgaggtgcga aagcgtgggt agcaaacggg 780
attagatacc ccggtagtcc acgccgtaaa cgatgtcaac taactgttgg gcgggtttcc 840
gcttagtggt gcastaacgc aataagttga ccgcctgggg agtacggccg caaggctaaa 900
actcaaatga attgacgggg gcccgcacaa gcggtggagc atgtggttta attcgatgca 960
acgcgaagaa ccttacctac ccttgacata cagagaactt tctagagata gattggtgcc 1020
ttcgggaact ctgatacagg tgctgcatgg ctgtcgtcag ctcgtgtcgt gagatgttgg 1080
gttaagtccc gtaacgagcg caacccttat ccttagttgc taccatttag ttgggcactc 1140
taaggagact gccggtgata aaccggagga aggtggggac gacgtcaagt catcatggcc 1200
cttatgggta gggctacaca cgtgctacaa tggccggtac agagggccgc aaactcgcga 1260
gagtaagcta atcccttaaa gccggtccta gtccggattg cagtctgcaa ctcgactgca 1320
tgaagctgga atcgctagta atcgcggatc agaatgccgc ggtgaattcg ttcccgggcc 1380
ttgtacacac cgcccgtcac accatgggag tgggttgcaa aagaagtggg taggctaacc 1440
ttcgggaggc cgctcaccac tttgtgattc atgactgggg tgaagtcgta acaaggtagc 1500
cctaggggaa cctggggctg gatcacctcc tt 1532

<210> 2
<211> 1528
<212> DNA
<213> Cycloclasticus pugetii

<220>
<221> rRNA
<222> (1)..(1528)
<223>

<400> 2
agagtttgat catggctcag attgaacgct ggcggcatgc ctaacacatg caagtcgaac 60

```
ggaaacgatg ctagcttgct agcaggcgtc gagtggcgga cgggtgagta atgcatagga    120
atctacctaa tagtgtggga caacctggtg aaaaccaggc taataccgca taatccctac    180
ggggcaaagc aggggacctt cgggccttgc gctaatagat gagcctatgt cggattagct    240
agttggtgag gtaatggctc accaaggcaa cgatccgtag ctggtttgag aggatgatca    300
gccacactgg gactgagaca cggcccagac tcctacggga ggcagcagtg gggaatattg    360
cacaatggag gaaactctga tgcagcaatg ccgcgtgtgt gaagaaggcc ttagggttgt    420
aaagcacttt cagtagggag gaaaagttta aggttaataa ccttaggccc tgacgttacc    480
tacagaagaa gcaccggcta actccgtgcc acagccggcg gtaatacgga gggtgcaagc    540
gttaatcgga attactgggc gtaaagcgcg cgtaggcggt taaacaagtc agatgtgaaa    600
gccccgggct caacctggga actgcatttg aaactgttta gctagagtgt ggtagaggag    660
agtggaattt caggtgtagc ggtgaaatgc gtagatatct gaaggaacac cagtggcgaa    720
ggcggctctc tggaccaaca ctgacgctga ggtgcgaaag cgtgggtagc aaacgggatt    780
agataccccg gtagtccacg ccgtaaacga tgtcaactga ctgttgggcg ggtttccgct    840
tagtggtgca staacgcaat aagttgaccg cctggggagt acggccgcaa ggctaaaact    900
caaatgaatt gacgggggcc cgcacaagcg gtggagcatg tggtttaatt cgatgcaacg    960
cgaagaacct tacctaccct tgacatacag agaactttct agagatagat tggtgccttc   1020
gggaactctg atacaggtgc tgcatggctg tcgtcagctc gtgtcgtgag atgttgggtt   1080
aagtcccgta acgagcgcaa cccttatcct tagttgctac catttagttg ggcactctaa   1140
ggagactgcc ggtgataaac cggaggaagg tggggacgac gtcaagtcat catggccctt   1200
atgggtaggg ctacacacgt gctacaatgg ccggtacaga gggccgcaaa ctcgcgagag   1260
taagctaatc ccttaaagcc ggtcctagtc cggattgcag tctgcaactc gactgcatga   1320
agctggaatc gctagtaatc gcggatcaga atgccgcggt gaattcgttc ccgggccttg   1380
tacacaccgc ccgtcacacc atgggagtgg gttgcaaaag aagtgggtag gctaacttcg   1440
ggaggccgct caccactttg tgattcatga ctggggtgaa gtcgtaacaa ggtagcccta   1500
ggggaacctg ggctggatc acctcctt                                       1528
```

<210> 3

<211> 1529

<212> DNA

<213> Cycloclasticus pugetii

<220>

<221> misc_feature

<222> (1100)..(1100)

<223> n

<220>
<221> rRNA
<222> (1)..(1529)
<223>

<220>
<221> misc_feature
<222> (1101)..(1101)
<223> n

<400> 3
agagtttgat catggctcag attgaacgct ggcggcatgc ctaacacatg caagtcgaac 60
ggaaacgatg ctagcttgct agcaggcgtc gagtggcgga cgggtgagta atgcatagga 120
atctacctaa cagtggggga caacctggtg aaaaccagsc taataccgca taatccctaa 180
cgggcaaagc aggggacctt cgggccttgc gctaatagat gagcctatgt cggattagct 240
agttggtgag gtaatggccc accaaggcaa cgatccgtag ctggtttgag aggatgatca 300
gccacactgg gactgagaca cggcccagac tcctacggga ggcagcagtg gggaatattg 360
cacaatggag gaaactctga tgcagcaatg ccgcgtgtgt gaagaaggcc ttagggttgt 420
aaagcacttt cagtagggag gaaaagttta aggttaataa ccttaggccc tgacgttacc 480
tacagaagaa gcaccggcta actccgtgcc agcagccgcg gtaatacgga ggggtgcaag 540
cgttaatcgg aattactggg cgtaaagcgc gcgtaggcgg ttaaacaagt cagatgtgaa 600
agccccgggc tcaacctggg aactgcattt gaaactgttt agctagagtg tggtagagga 660
gagtggaatt tcaggtgtag cggtgaaatg cgtagatatc tgaaggaaca ccagtggcga 720
aggcggctct ctggaccaac actgacgctg aggtgcgaaa gcgtgggtag caaacgggat 780
tagatacccc ggtagtccac gccgtaaacg atgtcaacta actgttgggc gggtttccgc 840
ttagtggtgc astaacgcaa taagttgacc gcctggggag tacggccgca aggctaaaac 900
tcaaatgaat tgacgggggc ccgcacaagc ggtggagcat gtggtttaat tcgatgcaac 960
gcgaagaacc ttacctaccc ttgacataca gagaactttc tagagataga ttggtgcctt 1020
cgggaactct gatacaggtg ctgcatggct gtcgtcagct cgtgtcgtga gatgttgggt 1080
taagtcccgt aacgagcgcn nycttatcct tagttgctac catttagttg ggcactctaa 1140
ggagactgcc ggtgataaac cggaggaagg tggggacgac gtcaagtcat catggccctt 1200
atgggtaggg ctacacacgt gctacaatgg ccggtacaga gggccgcaaa ctcgcgagag 1260
taagctaatc ccttaaagcc ggtcctagtc cggattgcag tctgcaactc gactgcatga 1320

agctggaatc gctagtaatc gcggatcaga atgccgcggt gaattcgttc ccgggccttg 1380
tacacaccgs ccgtcacacc atgggagtgg gttgcaaaag aagtgggtag gctaaccttc 1440
gggaggccgc tcaccacttt gtgattcatg actggggtga agtcgtaaca aggtagccct 1500
aggggaacct ggggctggat cacctcctt 1529

<210> 4
<211> 1526
<212> DNA
<213> Cylcoclasticus pugetii

<220>
<221> misc_feature
<222> (849)..(849)
<223> n

<220>
<221> rRNA
<222> (1)..(1526)
<223>

<400> 4
agagtttgat catggctcag attgaacgct ggcggcatgc taacacatgc aagtcgaacg 60
gaaacgatgc tagcttgcta caggcgtcga gtggcggacg ggtgagtaat gcataggaat 120
ctacctaata gtgggggaca acctggtgaa aaccagctaa taccgcataa tccctacggg 180
gcaaagcagg ggaccttcgg gccttgcgct aatagatgag cctatgtcgg attagctagt 240
tggtgaggta atggctcacc aaggcaacga tccgtagctg gtttgagagg atgatcagcc 300
acactgggac tgagacacgg cccagactcc tacgggaggc agcagtgggg aatattgcac 360
aatggaggaa actctgatgc agcaatgccg cgtgtgtgaa gaaggcctta gggttgtaaa 420
gcactttcag tagggaggaa aagtttaagg ttaataacct taggccctga cgttacctac 480
agaagaagca ccggctaact ccgtgccagc agccgcggta atacggaggg tgcaagcgtt 540
aatcggaatt actgggcgta aaagcgcgcg taggcggtta aacaagtcag atgtgaaagc 600
cccgggctca acttgggaac tgcatttgaa actgtttagc tagagtgtgg tagaggagag 660
tggaatttca ggtgtagcgg tgaaatgcgt agatatctga aggaacacca gtggcgaagg 720
cggctctctg gaccaacact gacgctgagg tgcgaaagcg tgggtagcaa acgggattag 780
ataccccggt agtccacgcc gtaaacgatg tcaactaact gttgggcggg tttccgctta 840

gtggtgcant aacgcaataa gttgaccgcc tggggagtac ggccgcaagg ctaaaactca 900
aatgaattga cgggggcccg cacaagcggt ggagcatgtg gtttaattcg atgcaacgcg 960
aagaacctta cctacccttg acatacagag aactttctag agatagattg gtgcttcggg 1020
aactctgata caggtgctgc atggctgtcg tcagctcgtg tcgtgagatg ttgggttaag 1080
tcccgtaacg agcgcaaccc ttatccttag ttgctaccat ttagttgggc actctaagga 1140
gactgccggt gataaaccgg aggaaggtgg ggacgacgtc aagtcatcat ggcccttatg 1200
ggtagggcta cacacgtgct acaatggccg gtacagaggg ccgcaaactc gcgagagtaa 1260
gctaatccct taaagccggt cctagtccgg attgcagtct gcaactcgac tgcatgaagc 1320
tggaatcgct agtaatcgcg gatcagaatg ccgcggtgaa ttcgttcccg ggccttgtac 1380
acaccgcccg tcacaccatg ggagtgggtt gcaaaagaag tgggtaggct aacttcggga 1440
ggccgctcac cactttgtga ttcatgactg gggtgaagtc gtaacaaggt agccctaggg 1500
gaacctgggg ctggatcacc tcctta 1526

<210> 5
<211> 18
<212> DNA
<213> Artificial Sequence

<220>
<223> Description of Artificial Seuqence: synthetic DNA

<400> 5
ggaaacccgc ccaacagt 18

<210> 6
<211> 20
<212> DNA
<213> Artificial Sequence

<220>
<223> Description of Artificial Sequence: synthetic DNA

<400> 6
tgcaccacta agcggaaacc 20

<210> 7
<211> 38
<212> DNA
<213> Artificial Sequence

<220>

<223> Description of Artificial Sequence: synthetic DNA

<400> 7
ggaaacccgc ccaacagttg caccactaag cggaaacc 38

<210> 8

<211> 20

<212> DNA

<213> Artificial Sequence

<220>

<223> Description of Artificial Sequence: synthetic DNA

<400> 8
agagtttgat cctggctcag 20

<210> 9

<211> 18

<212> DNA

<213> Artificial Sequence

<220>

<223> Description of Artificial Sequence: synthetic DNA

<400> 9
aaaggaggtg atccagcc 18